# EUROPÄISCHE PATENTSCHRIFT

(11) **EP 1 017 492 B1**
(45) Veröffentlichungstag und Bekanntmachung des Hinweises auf die Patenterteilung: **02.01.2003**
(21) Anmeldenummer: 98951329.6
(22) Anmeldetag: 29.08.1998
(51) Int. Cl.: B01J 31/02, B01J 31/24, C07C 45/50

(54) **VERFAHREN ZUR HERSTELLUNG VON ALDEHYDEN DURCH HYDROFORMULIERUNG**
METHOD FOR PREPARING ALDEHYDES BY HYDROFORMYLATION
PROCEDE DE PREPARATION D'ALDEHYDES PAR HYDROFORMYLATION

(30) Priorität: 16.09.1997 DE 19740672
(43) Veröffentlichungstag der Anmeldung: 12.07.2000
(73) Patentinhaber: Celanese Chemicals Europe GmbH, 60439 Frankfurt am Main (DE)
(72) Erfinder: BOGDANOVIC, Sandra, D-60322 Frankfurt am Main (DE)
(86) Internationale Anmeldenummer: EP9805491
(87) Internationale Veröffentlichungsnummer: WO99013982

(56) Entgegenhaltungen:
- EP-A- 0 068 499
- WO-A-95/22405
- WO-A-98/30526
- WO-A-98/30527
- FR-A- 2 291 960
- FR-A- 2 314 910
- US-A- 4 329 511

## Beschreibung

Die vorliegende Erfindung betrifft einen Katalysator sowie dessen Verwendung zur Hydroformylierung olefinisch ungesättigter Verbindungen mit 9 bis 18 Kohlenstoffatomen mit Wasserstoff und Kohlenmonoxid bei erhöhtem Druck.

Es ist bekannt, für die Katalyse von Additionsreaktionen an olefinisch ungesättigten Verbindungen Metalle der Gruppe VIII des Periodensystems der Elemente zu verwenden. Oftmals wird der Katalysator als im Reaktionsmedium unlöslicher Feststoff eingesetzt (heterogene Katalyse).

Eine unter dem Gesichtspunkt der industriellen Verwertbarkeit bedeutende katalytische Additionsreaktion an Olefine ist die Hydroformylierung.
Hierbei werden Aldehyde und Alkohole hergestellt durch Umsetzung von Olefinen mit Kohlenmonoxid und Wasserstoff, wobei die Aldehyde und Alkohole ein Kohlenstoffatom mehr als das Ausgangsolefin enthalten. Der verwendete Katalysator wird hierbei üblicherweise in homogener Phase mit dem Olefin eingesetzt. Die Reaktion wird vorzugsweise durch Hydridometallcarbonyle der Metalle der VIII. Gruppe des Periodensystems katalysiert. Neben Kobalt, das als Katalysatormetall in großem Umfang technische Anwendung findet, hat Rhodium vorzugsweise für die Hydroformylierung niederer Olefine zunehmende Bedeutung erlangt. Im Gegensatz zu Kobalt gestattet Rhodium, die Reaktion bei niedrigem Druck durchzuführen, darüber hinaus werden bei Einsatz endständiger Olefine bevorzugt geradkettige n-Aldehyde und nur in untergeordnetem Maße iso-Aldehyde gebildet. Darüber hinaus ist auch die Hydrierung der olefinischen Verbindungen zu gesättigten Kohlenwasserstoffen in Gegenwart von Rhodium-Katalysatoren deutlich niedriger als bei Anwendung von Kobalt-Katalysatoren.

Bei den in der Technik eingeführten Verfahren wird der Rhodium-Katalysator in Form von modifizierten Hydridorhodiumcarbonylen eingesetzt, die zusätzliche und gegebenenfalls im Überschuß eingesetzte Liganden enthalten. Besonders bewährt haben sich als Liganden tertiäre Phosphine oder Phosphite. Ihre Anwendung ermöglicht, den Reaktionsdruck auf Werte unter 30 MPa zu senken.
Probleme wirft bei diesem Verfahren jedoch die Abtrennung der Reaktionsprodukte und die Wiedergewinnung der im Reaktionsprodukt homogen gelösten Katalysatoren auf. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und Alkohole aber nur bei der Hydroformylierung kurzkettiger Olefine beschritten werden.

Unter der Bezeichnung "kurzkettige Olefine" werden hier und im folgenden Olefine mit höchstens 8 Kohlenstoffatomen im Molekül verstanden.

So offenbart US-A-4,151,209 ein Hydroformylierungsverfahren in Gegenwart eines Rhodium und Triorganophosphine enthaltenden Katalysators, der in einem inerten Lösungsmittel gelöst vorliegt. Als Lösungsmittel werden Polyglykole eingesetzt, die eine Molmasse von wenigstens 500 aufweisen. Nach dem offenbarten Verfahren liegt der Katalysator und der gebildete Aldehyd homogen gelöst, in dem Reaktionsgemisch vor, aus dem während der Hydroformylierungsreaktion durch kontinuierliches Strippen, mit einem Olefin, Wasserstoff und Kohlenmonoxid enthaltenden Gasgemisch der gewünschte Aldehyd abgetrennt wird. Bei diesem Verfahren werden Wertprodukt und Katalysatorphase einer thermischen Belastung ausgesetzt.

Bei der Hydroformylierung langkettiger Olefine oder olefinischer Verbindungen mit funktionellen Gruppen werden Produkte mit hohem Siedepunkt gebildet, die sich destillativ vom homogen gelösten Rhodium-Komplexkatalysator nicht abtrennen lassen. Die thermische Belastung des Destillationsgutes führt durch Dickölbildung zu erheblichen Verlusten an Wertprodukten und an Katalysator durch Zersetzung der Rhodiumkomplexverbindungen.
Unter der Bezeichnung "langkettige Olefine" werden hier und im folgenden Olefine mit mehr als 8 Kohlenstoffatomen im Molekül verstanden.

Das Problem der thermischen Zersetzung wird vermieden, wenn man eine Zweiphasenkatalyse verwendet werden. Hierbei liegen zwei flüssige, miteinander nicht mischbare Phasen vor, von denen die eine, organische Phase das Olefin enthält und die andere, zumeist polare Phase, den Katalysator enthält. Voraussetzung für die Anwendung dieses Verfahrens ist die Löslichkeit des Katalysators in der polaren Phase. Im industriellen Maßstab wird als polare Phase eine wäßrige Phase und als Katalysator eine Rhodiumkomplexverbindung verwendet. Die Löslichkeit des Katalysators in der wäßrigen Phase wird hierbei durch Verwendung sulfonierter Triarylphosphine als Komplexbestandteil erreicht. Die Abtrennung des Katalysators vom Reaktionsprodukt nach Beendigung der Hydroformylierungsreaktion erfolgt bei dieser Verfahrensvariante einfach durch Trennung von wäßriger und organischer Phase, d. h. ohne Destillation und damit ohne zusätzliche thermische Verfahrensschritte. Ein solches Verfahren ist beispielsweise in der DE-PS 26 27 354 beschrieben. Ein besonderes Merkmal dieser Arbeitsweise ist, daß aus endständigen Olefinen mit hoher Selektivität n-Aldehyde und nur in untergeordnetem Maße iso-Aldehyde (d.h. in α-Position zur Aldehydgruppe verzweigte Aldehyde) gebildet werden. Neben sulfonierten Triarylphosphinen werden als Komplexbestandteile wasserlöslicher Rhodiumkomplexverbindungen auch carboxylierte Triarylphosphine eingesetzt.

Der Einsatz wasserlöslicher Katalysatoren hat sich auch bei der Hydroformylierung niederer Olefine, insbesondere Propen und Buten, bewährt. Setzt man höhere Olefine wie Penten oder Hexen ein, geht jedoch die Umsetzungsgeschwindigkeit bereits merklich zurück. Die Wirtschaftlichkeit der Umsetzung in technischem Maßstab ist bei Einsatz von Olefinen mit mehr als vier Kohlenstoffatomen häufig nicht mehr in gewünschtem Umfang gegeben.
Um bei der Hydroformylierung höherer Olefine mittels wasserlöslicher Katalysatoren den Umsatz und/oder die Selektivität der Reaktion zu n-Aldehyden zu steigern, wurden auch spezielle amphiphile Reagenzien oder Lösungsvermittler eingesetzt.
Der Zusatz dieser Stoffe führt dazu, daß der Stofftransport zwischen den einzelnen Phasen und damit die Mischbarkeit von wäßriger Katalysatorphase und organischer Phase begünstigt werden.

So wird in der DE 31 35 127 A1 die Hydroformylierung von Olefinen unter Verwendung Verwendung amphiphiler Reagenzien beschrieben. Die Tabelle 7 zeigt, daß die Hydroformylierung von 1-Dodecen mittels Rhodium und monosulfoniertem Triphenylphosphin (3-Ph₂PC₆H₄SO₃Na) ohne Zusatz eines amphiphilen Reagenz zu einem Umsatz von 56 % (Beispiel 77) führt, während der Zusatz eines speziellen, langkettigen Alkylethoxylats der Formel C₁₂H₂₅(OCH₂CH₂)₂₃OH (vertrieben von der Firma ICI Chemicals unter der Bezeichnung Brij 35®) zu einer Minderung des Umsatzes auf 37 % (Beispiel 78) führt.

Die DE 34 12 335 betrifft ebenfalls die Hydroformylierung von Olefinen unter Verwendung quaternärer Ammoniumsalze. Wie aus Tabelle 4 hervorgeht, führt die Hydroformylierung von Hexen mittels Rhodium und Trinatrium-tri(msulfophenyl)phosphin ohne Zusatz eines Lösungsvermittlers zu einem Umsatz von 36 % (Beispiel 10), während ein Zusatz von 2,5 % Triethylenglykol (Beispiel 14) bzw. von 5 % Polyglykol 200 (Beispiel 11) nur einen Umsatz von 43,5 % bzw. 43 % bewirkt. Ein sehr hoher Umsatz, nämlich 86 %, wird hingegen durch Zusatz von 2,5 % Trimethylhexadecylammoniumbromid als Lösungsvermittler erzielt.
Diese Druckschrift zeigt, daß erst der Zusatz quaternärer Ammoniumsalze eine beachtliche Steigerung des Umsatzes zur Folge hat. Dementgegen hat weder der Zusatz von Tri- oder Polyglykolen, noch eine Erhöhung der Menge dieser Stoffe um das Doppelte (von 2,5 auf 5 %), eine signifikante Steigerung des Umsatzes zur Folge.

Ein Nachteil bei der Verwendung quaternärer Ammoniumsalze als amphiphile Reagenzien liegt allerdings in ihrer schlechten biologischen Abbaubarkeit. So führt beispielsweise die Anwesenheit quaternärer Ammoniumsalze im Abwasser zu erheblichen Schwierigkeiten bei der Abwasseraufbereitung.

Ein weiterer Nachteil bei der Verwendung amphiphiler Reagenzien und Lösungsvermittler liegt darin, daß die mit diesen Verbindungen erreichte Steigerung der Vermischbarkeit von wäßriger Katalysatorphase und organischer Phase mit einer erhöhten Löslichkeit von organischer Phase in wäßriger Phase und von wäßriger Phase in organischer Phase einhergeht. Auf diese Weise kann in zunehmendem Maße sowohl amphiphiles Reagenz und Lösungsvermittler als auch Rhodium und wasserlösliches Phosphin in die organische Phase gelangen und nach Phasentrennung mit der organischen Phase ausgetragen werden.
Der Austrag dieser Substanzen über die organische Phase ist natürlich unerwünscht, da im gleichen Maße neue Substanzen wieder zur wäßrigen Phase hinzugegeben werden müssen, was insbesondere in Hinblick auf Rhodium mit einem erheblichen finanziellen Mehraufwand verbunden ist.

Des weiteren findet mit höherem Zusatz amphiphiler Reagenzien oder Lösungsvermittler - d.h. mit steigender Vermischbarkeit von wäßriger Katalysatorphase und organischer Phase - die für die Phasentrennung erforderliche Entmischung infolge der Bildung von Emulsionen oder Lösungen nicht mehr oder nicht in ungenügendem Umfang statt. Dies ist insbesondere bei solchen amphiphilen Reagenzien der Fall, die auch als Tenside oder Schaumbildner eingesetzt werden können.
Dies ist insofern von Nachteil, als eine gute Entmischbarkeit eine unabdingbare Voraussetzung für die erforderliche, die Hydroformylierung abschließende Trennung von organischer und wäßriger Phase ist.

Die Aufgabe der Erfindung besteht in der Bereitstellung eines Katalysators für Additiionsreaktionen an olefinisch ungesättigte Verbindungen, sowie dessen Verwendung, insbesondere zur Herstellung längerkettiger Aldehyde durch Hydroformylierung von Oelfinen. Die Verwendung dieses Katalysators soll höhere Umsätze im Vergleich zum Stand der Technik ermöglichen, die Nachteile des Standes der Technik vermeiden und sich auf einfache Weise im großtechnischen Maßstab realisieren lassen.

Diese Aufgabe wird gelöst durch einen Katalysator, enthaltend
(a) mehr als 70 Gew.-% eines Polyethylenglykols der Formel (I) 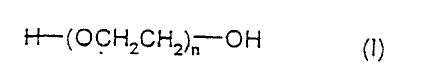 in der
   - n eine ganze Zahl zwischen 3 und 16 darstellt; und
   - das zahlenmittlere Molekulargewicht weniger als 650 beträgt.
(b) Rhodium in elementarer oder gebundener Form;
(c) 2 bis 25 Gew.-% eines Liganden, der ein wasserlösliches, sulfoniertes Triarylphosphin ist;
(d) höchstens 25 Gew.-% Wasser.

Der erfindungsgemäße Katalysator eignet sich besonders für die Katalyse von Carbonylierungen oder Hydrierungen von olefinisch ungesättigten Verbindungen, wie beispielsweise Alkoxycarbonylierungen, Hydroxycarbonylierungen oder Hydrosulfinierungen, insbesondere zur Hydroformylierung.

Der erfindungsgemäße Katalysator zeichnet sich insbesondere dadurch aus, daß trotz der sehr hohen Konzentration des Polyethylenglykols der Formel (I) kein signifikanter Anstieg von Rhodium oder Liganden in der organischen Phase und somit kein erhöhter Austrag des Katalysators über die organische Phase beobachtet wird. Darüber hinaus ist die Entmischbarkeit von organischer Phase und wäßriger Katalysatorphase so hoch, daß eine schnelle Trennung von organischer Phase und wäßriger Phase gewährleistet ist. Bei Verwendung dieses Katalysators werden weder schwertrennbare Emulsionen noch nicht trennbare, homogene Lösungen gebildet. Diese Eigenschaften des erfindungsgemäßen Katalysators sind insoweit überraschend, weil Polyethylenglykole der Formel (I) gemäß dem Stand der Technik üblicherweise als Lösungsvermittler eingesetzt werden.

In einer bevorzugten Ausführungsform der Erfindung enthält der Katalysator höchstens 95 Gew.-% des Polyethylenglykols der Formel (I). Insbesondere kann der 80 bis 85 Gew.-% des Polyethylenglykols der Formel (I) enthalten. Besonders für den erfindungsgemäßen Katalysator geeignet sind Polyethylenglykole mit einem zahlenmittleren Molekulargewicht zwischen 150 und 200.

Beispiele für Verbindungen der Formel (I) sind Polyethylenglykole mit einem mittleren Molgewicht von ungefähr 200 (PEG 200), 400 (PEG 400) oder 600 (PEG 600);
Bei diesen Polyethylenglykolen bedeutet die Bezeichnung
- PEG 200 ein Gemisch von Polyethylenglykolen der Formel H(OCH₂CH₂)ₙOH, in der n für eine ganze Zahl von 3 bis 6 steht;
- PEG 400 ein Gemisch von Polyethylenglykolen der Formel H(OCH₂CH₂)ₙOH, in der n für eine ganze Zahl von 7 bis 10 steht;
- PEG 600 ein Gemisch von Polyethylenglykolen der Formel H(OCH₂CH₂)ₙOH, in der n für eine ganze Zahl von 11 bis 16 steht; und
Diesen Gemischen ist jeweils ein entsprechendes mittleres Molgewicht von etwa 200 (PEG 200), etwa 400 (PEG 400) respektive etwa 600 (PEG 600) zuzuordnen.

Bei dem Liganden handelt es sich vorzugsweise um ein wasserlösliches organisches Phosphin.
Für das erfindungsgemäße Verfahren besonders gut bewährt haben sich trisulfonierte Triarylphosphine der Formel (II) 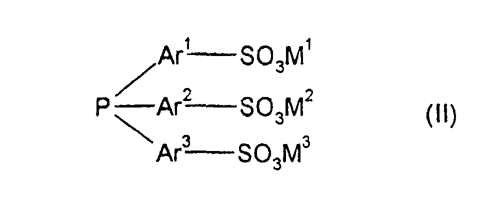 in der
- Ar¹, Ar² und Ar³ unabhängig voneinander jeweils einen Phenyl-, Naphtyl-, Biphenyl-, Phenylnaphtyl- oder Binaphtylrest darstellen;
- M¹, M² und M³ unabhängig voneinander jeweils ein Alkalimetallion oder ein Ammoniumion darstellen.

Es ist aber auch möglich, daß M¹, M² und M³ andere, höherwertige Kationen darstellen wie beispielsweise Erdalkali- oder Zinkkationen, wobei der Ladungsausgleich maßgeblich die Anzahl dieser Kationen bestimmt.

Dieses sulfonierte Triarylphosphin der Formel (II) ist gemäß einer bevorzugten Ausführungsform der vorliegenden Erfindung ein trisulfoniertes Triarylphosphin, insbesondere Trinatrium-tri(m-sulfophenyl)phosphin der Formel

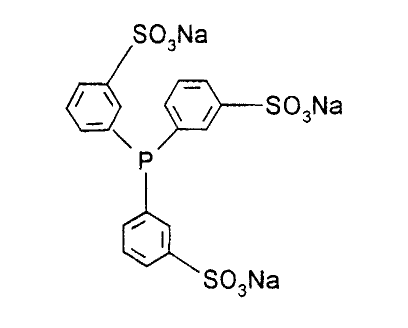

Dieses Trinatriumsalz enthält aufgrund seiner Herstellung durch Sulfonierung von Triphenylphosphin auch noch Anteile an mono- und disulfonierten Verbindungen und geringe Anteile der entsprechenden Phosphinoxide.

Bei Verwendung von Liganden mit mindestens einem Phosphoratom, insbesondere Liganden der Formel (II) hat es sich als günstig erwiesen, daß der Katalysator 10 bis 1.000 ppm, vorzugsweise 200 bis 500 ppm, insbesondere 300 bis 400 ppm, Rhodium enthält.
Das Verhältnis von Rhodium zum Liganden kann hierbei zwischen 1 : 10 und 1 : 1000, vorzugsweise zwischen 1 : 50 und 1 : 200, liegen.

Das organische Phosphin kann auch ein sulfoniertes Triarylphosphin mit zwei Phosphoratomen sein, das beispielsweise einen Rest -(CH₂)ₓ-Ar-Ar-(CH₂)ₓ- enthält, worin
- x für eine ganze Zahl von 1 bis 4, insbesondere 1 bis 2, vorzugsweise 1 steht;
- Ar-Ar Biphenyl oder Binaphthyl bedeutet;
- die -(CH₂)ₓ-Gruppe mit der einen Bindung jeweils in ortho-Stellung zu der die beiden Arylreste verbindenden Aryl-Arylbindung Ar-Ar steht, und mit der anderen Bindung jeweils mit einem Phosphoratom, das jeweils zwei weitere, gleiche oder verschiedene Arylreste, insbesondere Phenylreste besitzt, verbunden ist.

Beispiele für derartige, zwei Phosphoratome enthaltende, sulfonierte Triarylphosphine sind Verbindungen der Formel (III) 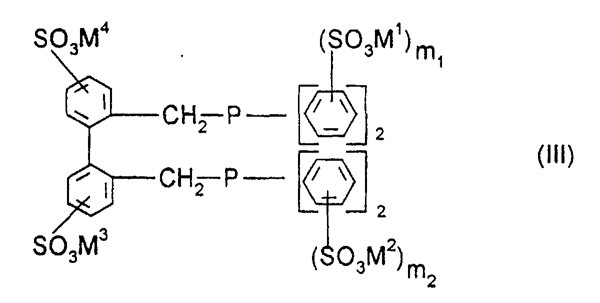 in der
- m¹ und m² den Wert 0 oder 1 haben können, wobei die Summe von m¹ und m² mindestens 1 beträgt; und
- M¹, M², M³ und M⁴ unabhängig voneinander jeweils ein Alkalimetallion oder ein Ammoniumion darstellen.

Es ist aber auch möglich, daß M¹, M², M³ und M⁴ andere, höherwertige Kationen darstellen wie beispielsweise Erdalkali- oder Zinkkationen, wobei der Ladungsausgleich maßgeblich die Anzahl dieser Kationen bestimmt.

Ebensogut kann das organische, zwei Phosphoratome enthaltende Phosphin eine Verbindung der Formel (IV) 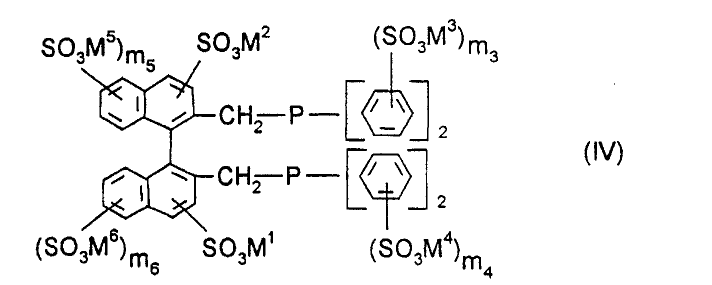 sein, in der
- m₃, m₄, m₅ und m₆ den Wert 0 oder 1 haben können, wobei die Summe von m₃, m₄, m₅ und m₆ mindestens 2 beträgt; und
- M¹, M², M³, M⁴, M⁵ und M⁶ unabhängig voneinander jeweils ein Alkalimetallion oder ein Ammoniumion darstellen.

Auch hier ist möglich, daß M¹, M², M³, M⁴, M⁵ und M⁶ andere, höherwertige Kationen darstellen wie beispielsweise Erdalkali- oder Zinkkationen, wobei der Ladungsausgleich maßgeblich die Anzahl dieser Kationen bestimmt.

Bei Verwendung von Liganden der Formeln (III) und (IV) hat es sich als günstig erwiesen, daß der Katalysator 20 bis 500 ppm, vorzugsweise 30 bis 150 ppm, insbesondere 40 bis 100 ppm, Rhodium enthält.
Das Verhältnis von Rhodium zum Liganden kann hierbei zwischen 1 : 5 und 1 : 100, vorzugsweise zwischen 1 : 5 und 1 : 50, insbesondere zwischen 1 : 8 und 1 : 15, liegen.
Solche zwei Phosphoratome enthaltenden Triarylphosphine der Formeln (III) und (IV) weisen insbesondere vier bis acht -SO₃M Reste, auf. Die -SO₃M Reste sitzen üblicherweise an den Arylresten des Restes -(CH₂)ₓ-Ar-Ar-(CH₂)ₓ- und an den zwei weiteren Arylresten, die mit dem Phosphor verbunden sind.

Alternativ können anstelle sulfonierter Triarylphosphine andere Triarylphosphine als Liganden eingesetzt werden, bei denen die SO₃M-Gruppe durch andere Gruppen ersetzt wird, die eine Wasserlöslichkeit des Triarylphosphins bewirken, wie z.B. PO₃M₂-Gruppen.

Der Katalysator kann gemäß einer besonders bevorzugten Ausführungsform zur Herstellung längerkettiger Aldehyde durch Hydroformylierung eines Olefins mit 9 bis 18 Kohlenstoffatomen, insbesondere mit 12 bis 14 Kohlenstoffatomen, verwendet werden.
Das Olefin kann aus aliphatischen, cycloaliphatischen und araliphatischen Olefinen, insbesondere aus aliphatischen und cycloaliphatischen α-Olefinen, ausgewählt sein. Die olefinische Verbindung kann eine oder mehrere Kohlenstoff-Kohlenstoff-Doppelbindung enthalten. Die Kohlenstoff-Kohlenstoff-Doppelbindung kann endständig oder innenständig angeordnet sein. Bevorzugt sind olefinische Verbindungen mit endständiger Kohlenstoff-Kohlenstoff-Doppelbindung.
Beispiele für α-olefinische Verbindungen (mit endständiger Kohlenstoff-Kohlenstoff-Doppelbindung) sind 1-Alkene, Alkylalkenoate, Alkylenalkanoate, Alkenylalkylether und Alkenole.
Ohne Anspruch auf Vollständigkeit seien als α-olefinische Verbindungen 1-Dodecen, 1-Tetradecen, 1-Hexadecen, 1-Octadecen genannt.
Als weitere Beispiele geeigneter olefinischer Verbindungen seien Diisobutylen, Tripropylen, Octol oder Dimersol (Dimerisierungsprodukte von Butenen), Tetrapropylen, acyclische, cyclische oder bicyclische Terpene, wie Myrcen, Limonen und Pinen erwähnt.
Im Hinblick auf einen industriellen Einsatz dieses Verfahrens ist das Olefin insbesondere ausgewählt aus der Gruppe von 1-Nonen, 1- Decen, 1- Undecen, 1-Dodecen, 1-Tetradecen, 1-Hexadecen und 1-Octadecen.

In einer besonders bevorzugten Ausführungsform des erfindungsgemäßen Verfahrens liegt das Olefin unter Reaktionsbedingungen in einer flüssigen, mit Wasser nicht mischbaren Phase vor.
Die den Katalysator enthaltende wäßrige Phase läßt sich auf vergleichsweise einfache Art herstellen, indem man ein wasserlösliches Rhodiumsalz, die sulfonierten Triarylphosphine und die Verbindung der Formel (I) in Wasser löst.
Geeignete Rhodiumsalze sind, ohne Anspruch auf Vollständigkeit zu erheben: Rhodium(III)sulfat, Rhodium(III)nitrat, Rhodium(III)carboxylate wie Rhodium(III)acetat, Rhodiumpropionat, Rhodiumbutyrat und Rhodium-2-ethylhexanoat.

Man kann die wäßrige Phase unmittelbar in die Hydroformylierung einsetzen oder sie zuvor einer Präformierung des Katalysators unter Reaktionsbedingungen unterwerfen, um sie anschließend in präformierter Form zu verwenden.
Die den Katalysator enthaltende Phase kann vorzugsweise entsprechend einer Menge von 2 x 10⁻⁶ bis 5 x 10⁻² Mol Rhodium pro Mol olefinischer Verbindung eingesetzt werden.

Der Druck während der Reaktion liegt im allgemeinen zwischen 20 und 150 bar, vorzugsweise zwischen 30 und 80 bar; Kohlenmonoxid und Wasserstoff können mit einem Druck von 5 bis 30 MPa, vorzugsweise von 10 bis 18 MPa, zugesetzt werden. Das Verhältnis von Kohlenmonoxid und Wasserstoff kann in weiten Grenzen variieren. Günstig ist ein Verhältnis von Kohlenmonoxid zu Wasserstoff von 10 : 1 bis 1 : 30, insbesondere von 5:1 bis 1:8, besonders bevorzugt von 1:2 bis 1:5. Ebenfalls vorteilhaft ist ein Zupressen von Synthesegas im Verhältnis Kohlenmonoxid zu Wasserstoff von 1:1 bis 1:5, insbesondere von 1 : 1 bis 1:3 und gegebenenfalls späteres Zupressen von reinem Wasserstoff im Verlauf der Reaktion.
Die Temperatur liegt üblicherweise zwischen 50 und 150 °C, vorzugsweise zwischen 100 und 140 °C.

Als Reaktionsgefäße werden Druckreaktoren mit magnetischer oder mechanischer Rühr- oder Mischeinrichtung verwendet. Während der Umsetzung ist eine gute Durchmischung der vorhandenen Phasen, d.h. von polarer Phase, Kohlenmonoxid/Wasserstoff und ggf. organischer Phase sicherzustellen. Dies kann insbesondere durch intensives Rühren und/oder Umpumpen von organischer und wäßriger Phase bewirkt werden.
Eine kontinuierliche Führung des Versuches ist ebenfalls möglich.

Nach dem Ende der Reaktion wird der Druckreaktor gekühlt, durch Druckentspannung von Kohlenmonoxid und Wasserstoff befreit und das Reaktionsgemisch entnommen. Bei Abschalten der Durchmischungseinrichtung trennen sich die Phasen von allein innerhalb von Sekunden. Die organische Phase kann destillativ aufgearbeitet werden und dann bei Bedarf gaschromatographisch untersucht werden.
Die folgenden Beispiele dienen der Erläuterung der Erfindung.

### Beispiel 1 (Vergleichsbeispiel):

### a): Herstellung der Katalysatorphase und Präformierung

30 mg (0,166 mmol) Rhodium(III)acetat werden in 19,5 ml einer 0,6 M wäßrigen Lösung des Trinatriumsalzes von TPPTS (Tris-(metasulfonato)triphenylphosphin) entsprechend einem Molverhältnis von Rhodium zu Ligand von 1 : 100 und 10,5 ml entgastem, destillierten Wasser gelöst und in einen 200 ml Stahlautoklav unter Stickstoffstrom gegeben. Die so hergestellte Katalysatorlösung wird bei einem Synthesegasdruck von 25 bar (CO/H₂ = 1/1) 3 Stunden lang auf 125 °C erwärmt, wobei der aktive Katalysatorkomplex entsteht. Die Lösung färbt sich intensiv gelb.

### b): Hydroformylierung

Zu der präformierten Katalysatorlösung aus 1.a) werden 120 mmol 1-Nonen unter einem Reaktionsdruck von 30 bar und bei 125 °C über einen vorgeschalteten 200 ml Stahlautoklav mit leichtem Überdruck zugegeben. Das Verhältnis von Olefin zu Rhodium beträgt 1030:1. Die Hydroformylierungsreaktion wird durch Einschalten des Magnetrührers in Gang gesetzt. Während einer Reaktionszeit von 3 Stunden wird die Temperatur auf 125 °C gehalten und der Reaktionsdruck in einem Druckband von +/- 3 bar durch manuelles Zudosieren von Synthesegas konstant gehalten. Nach Ablauf von 3 Stunden werden Rührer und Heizung abgeschaltet, der Autoklav auf 40 bis 50 °C abgekühlt und die obere Produktphase von der Katalysatorphase mittels Phasentrennung in einem Scheidetrichter getrennt. Produktphase und Katalysatorphase werden gewogen. Die Zusammensetzung der Produktphase wird mittels Gaschromatographie und ¹H-NMR-Spektroskopie ermittelt und aus der Zusammensetzung die Ausbeute an Hydroformylierungsprodukten und das Verhältnis von n-Decanal zu iso-Decanal bestimmt. Die Ausbeute an Hydroformylierungsprodukten beträgt 4,3 % das n/iso-Verhältnis beträgt 96 : 4.

### Beispiel 2:

Man verfährt wie in Beispiel 1, jedoch mit dem Unterschied, daß die Katalysatorphase aus 30 mg Rhodium(III)acetat, 19,5 ml einer 0,6 M wäßrigen Lösung des Trinatriumsalzes von TPPTS (Tris-(metasulfonato)triphenylphosphin) - entsprechend einem Molverhältnis Rhodium zu Ligand von 1 : 100 - und 50 ml PEG 150 angesetzt wird und aus diesem Gemisch insgesamt 15,7 ml Wasser bei reduziertem Druck abdestilliert werden. Die Katalysatorphase enthält somit 89,5 Gew.-% PEG 150.

### Beispiele 3 bis 16:

Die Vergleichsbeispiele 3, 6 und 14 werden analog zu Beispiel 1 durchgeführt. Die erfindungsgemäßen Beispiele 4, 5, 7 bis 13, 15 und 16 werden analog zu Beispiel 2 durchgeführt. In allen Beispielen werden 30 mg (0,166 mmol) Rhodium(III)acetat, 19,5 ml einer 0,6 M wäßrigen Lösung des Trinatriumsalzes von TPPTS (Tris-(metasulfonato)triphenylphosphin) entsprechend einem Molverhältnis von Rhodium zu Ligand von 1 : 100 und 120 mmol des entsprechenden Olefins eingesetzt. Das Verhältnis von Olefin zu Rhodium beträgt in allen Beispielen 1030 : 1.
Die weiteren Edukte und deren Mengen sowie die Reaktionsergebnisse sind in Tabelle 1 angegeben.

In der Tabelle 1 werden folgende Bezeichnungen verwendet:
- "Olefin" bezeichnet das verwendete Olefin; Rh steht für Rhodium(III)acetat;
- "H₂O abdest." steht für die Menge an abdestilliertem, aus der Katalysatorphase stammendem Wasser in ml;
- "PEG" steht für das verwendete Polyethylenglykol; hierbei steht die in der Tabelle angegebene Zahl für das zahlenmittlere Molekulargewicht des jeweiligen Polyethylenglykols. Die Menge des eingesetzen Polyethylenglykols beträgt in den Beispiele 4, 5, 7 bis 13, 15 und 16 jeweils 50 ml.
- "Anteil" steht für die Menge an Polyethylenglykol in der Katalysatorphase in Gew.-%;
- "t" steht für die Reaktionszeit in Minuten;
- "n : iso" steht für Verhältnis von n-Aldehyden zu iso-Aldehyden; "n.b." (nicht bestimmbar) bedeutet hierbei, daß sich das Verhältnis aufgrund des geringen Umsatzes nicht bestimmen läßt.

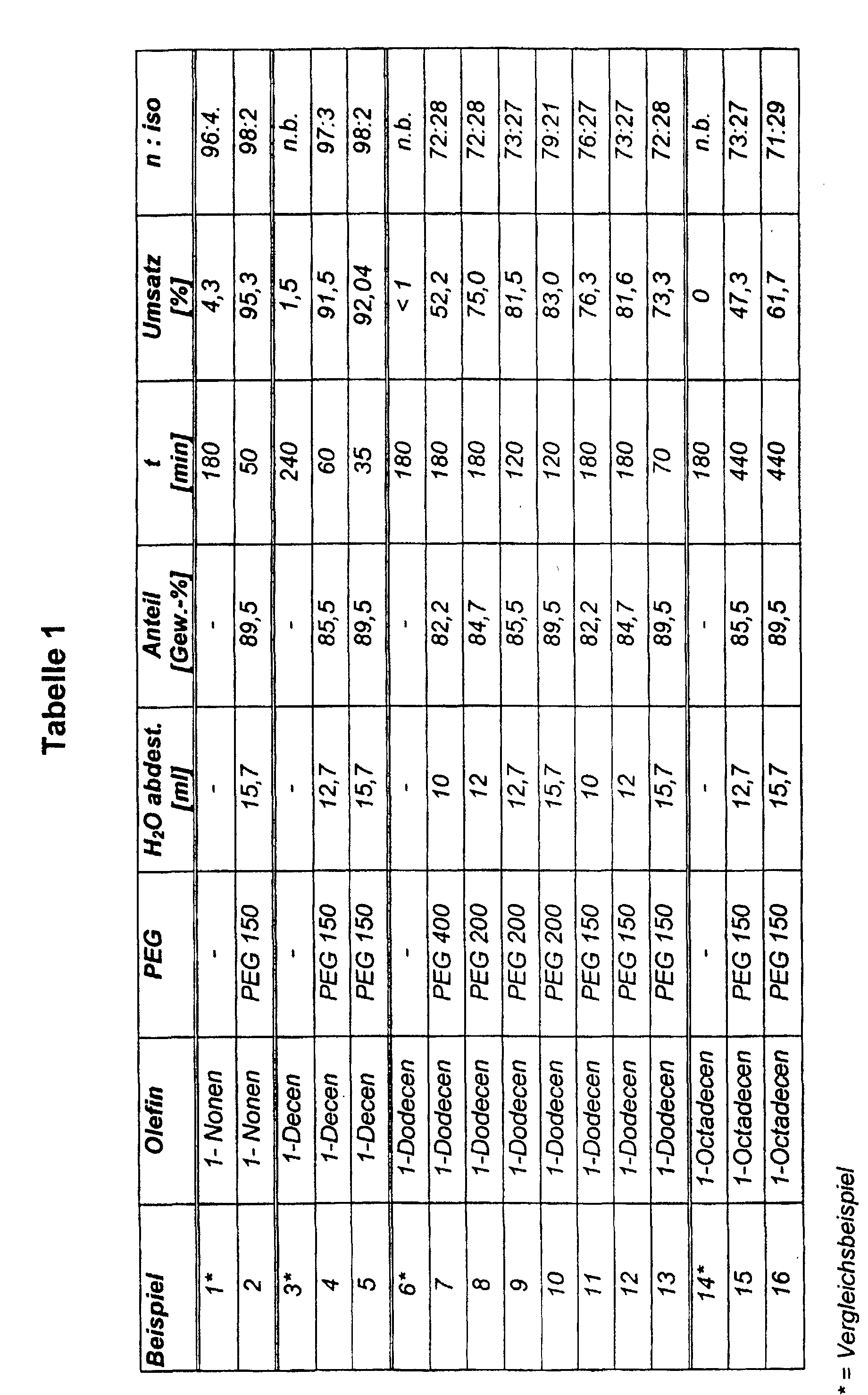

## Patentansprüche

1. Katalysator, enthaltend
(a) mehr als 70 Gew.-% eines Polyethylenglykols der Formel (I) 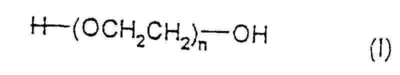 in der
• n eine ganze Zahl zwischen 3 und 16 darstellt; und
• das zahlenmittlere Molgewicht weniger als 650 beträgt.
(b) Rhodium in elementarer oder gebundener Form;
(c) 2 bis 25 Gew.-% eines Liganden, der ein wasserlösliches, sulfoniertes Triarylphosphin ist;
(d) höchstens 25 Gew.-% Wasser.

2. Katalysator nach Anspruch 1, **dadurch gekennzeichnet, daß** der Katalysator höchstens 95 Gew.-% des Polyethylenglykol der Formel (I) enthält.

3. Katalysator nach Anspruch 1 oder 2, **dadurch gekennzeichnet, daß** der Katalysator 80 bis 85 Gew.-% des Polyethylenglykols der Formel (1) enthält.

4. Katalysator nach Anspruch 1, **dadurch gekennzeichnet, daß** das wasserlösliche sulfonierte Triarylphosphin ein sulfoniertes Triarylphosphin der Formel (II) 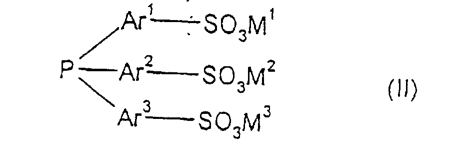 ist, in der
• Ar¹, Ar² und Ar³ unabhängig voneinander jeweils einen Phenyl-, Naphtyl-, Biphenyl-, Phenylnaphtyl- oder Binaphtylrest darstellen;
• M¹, M² und M³ unabhängig voneinander jeweils ein Alkalimetallion oder ein Ammoniumion darstellen.

5. Katalysator nach Anspruch 4, **dadurch gekennzeichnet, daß** das sulfonierte Triarylphosphin ein trisulfoniertes Triarylphosphin ist, insbesondere Trinatriumtri(m-sulfophenyl)phosphin.

6. Katalysator nach einem der vorherigen Ansprüche, **dadurch gekennzeichnet, daß** der Katalysator 100 bis 1.000 ppm, vorzugsweise 200 bis 500 ppm, insbesondere 300 bis 400 ppm, Rhodium enthält.

7. Katalysator nach einem der vorherigen Ansprüche, **dadurch gekennzeichnet, daß** das Verhältnis von Rhodium zum Liganden zwischen 1 : 10 und 1 : 1000, vorzugsweise zwischen 1 : 50 und 1 : 200, liegt.

8. Katalysator nach Anspruch 1, **dadurch gekennzeichnet, daß** das wasserlösliche sulfonierte Triarylphosphin eine Verbindung der Formel (III) 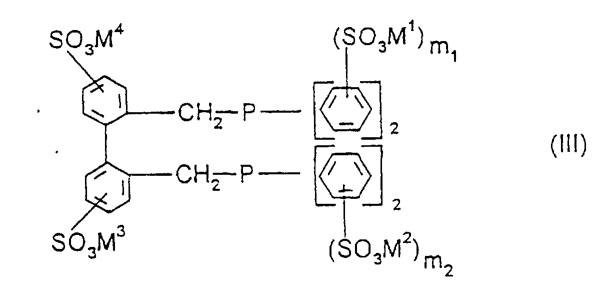 ist, in der
• m¹ und m² den Wert 0 oder 1 haben können, wobei die Summe von m¹ und m² mindestens 1 beträgt; und
• M¹, M², M³ und M⁴ unabhängig voneinander jeweils ein Alkalimetallion oder ein Ammoniumion darstellen.

9. Katalysator nach Anspruch 1, **dadurch gekennzeichnet, daß** das wasserlösliche sulfonierte Triarylphosphin eine Verbindung der Formel (IV) 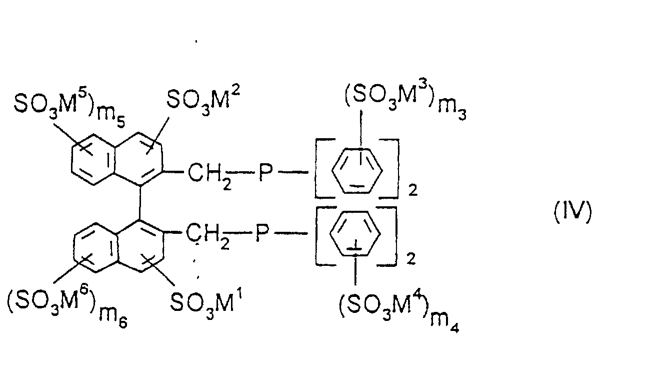 ist, in der
• m₃, m₄, m₅ und m₆ den Wert 0 oder 1 haben können, wobei die Summe von m₃, m₄, m₅ und m₆ mindestens 2 beträgt; und
• M¹, M², M³, M⁴, M⁵ und M⁶ unabhängig voneinander jeweils ein Alkalimetallion oder Ammoniumion darstellen.

10. Katalysator nach Anspruch 8 oder 9, **dadurch gekennzeichnet, daß** er 20 bis 500 ppm, vorzugsweise 30 bis 150 ppm, insbesondere 40 bis 100 ppm, Rhodium enthält.

11. Katalysator nach einem der Ansprüche 8 bis 10, **dadurch gekennzeichnet, daß** das Verhältnis von Rhodium zum Liganden zwischen 1 : 5 und 1 : 100, vorzugsweise zwischen 1 : 5 und 1 : 50, insbesondere zwischen 1 : 8 und 1 : 15 liegt.

12. Verfahren zur Herstellung von Aldehyden, **dadurch gekennzeichnet, daß** man ein C₉-C₁₆-Olefin mit Wasserstoff und Kohlenmonoxid in Gegenwart eines Katalysators nach einem der Ansprüche 1 bis 11 umsetzt.

13. Verfahren nach Anspruch 12, **dadurch gekennzeichnet, daß** das Olefin ausgewählt ist aus aliphatischen, cycloaliphatischen und araliphathischen Olefinen, insbesondere aus aliphatischen α-Olefinen und cycloaliphatischen Olefinen.

14. Verfahren nach Anspruch 12 oder 13, **dadurch gekennzeichnet, daß** das Olefin ausgewählt ist aus der Gruppe von 1-Nonen, 1-Decen, 1-Undecen, 1-Dodecen, 1-Tetradecen, 1-Hexadecen und 1-Octadecen.

15. Verfahren nach einem der Ansprüche 12 bis 14, **dadurch gekennzeichnet, daß** das Olefin unter Reaktionsbedingungen in einer flüssigen, mit dem Katalysator nicht mischbaren Phase vorliegt.

16. Verfahren nach einem der Ansprüche 12 bis 15, **dadurch gekennzeichnet, daß** der Katalysator entsprechend einer Menge von 2 x 10⁻⁶ bis 5 x 10⁻² Mol Rhodium pro Mol olefinischer Verbindung eingesetzt wird.

17. Verfahren nach einem der Ansprüche 12 bis 16, **dadurch gekennzeichnet, daß** der Druck während der Reaktion zwischen 20 und 150 bar, vorzugsweise zwischen 30 und 80 bar, liegt.

18. Verfahren nach einem der Ansprüche 12 bis 17, **dadurch gekennzeichnet, daß** der Temperatur während der Reaktion zwischen 50 und 150°C, vorzugsweise zwischen 100 und 140°C, liegt.

## Claims

1. A catalyst comprising
(a) more than 70% by weight of a polyethylene glycol of the formula (I) 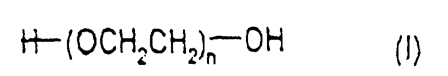 where
• n is an integer from 3 to 16; and
• the number average molecular weight is less than 650;
(b) rhodium in elemental or bound form;
(c) from 2 to 25% by weight of a ligand, which is a water-soluble, sulfonated triarylphosphine;
(d) not more than 25% by weight of water.

2. A catalyst as claimed in claim 1 which contains not more than 95% by weight of the polyethylene glycol of the formula (I).

3. A catalyst as claimed in claim 1 or 2 which contains from 80 to 85% by weight of the polyethylene glycol of the formula (I).

4. A catalyst as claimed in claim 1, wherein the water-soluble sulfonated triarylphosphine is a sulfonated triarylphosphine of the formula (II) 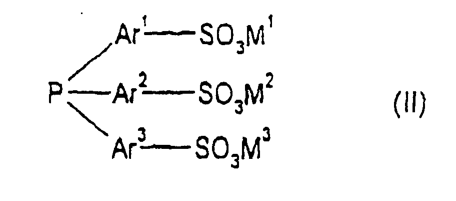 where
• Ar¹, Ar² and Ar³ are each, independently of one another, a phenyl, naphthyl, biphenyl, phenylnaphthyl or binaphthyl radical;
• M¹, M² and M³ are each, independently of one another, an alkaline metal ion or an ammonium ion.

5. A catalyst as claimed in claim 4, wherein the sulfonated triarylphosphine is a trisulfonated triarylphosphine, in particular trisodium tri(m-sulfophenyl)phosphine.

6. A catalyst as claimed in any one of the preceding claims which contains from 100 to 1000 ppm, preferably from 200 to 500 ppm, in particular from 300 to 400 ppm, of rhodium.

7. A catalyst as claimed in any one of the preceding claims, wherein the ratio of rhodium to the ligand is from 1 : 10 to 1 : 1000, preferably from 1 : 50 to 1 : 200.

8. A catalyst as claimed in claim 1, wherein the water-soluble sulfonated triarylphosphine is a compound of the formula (III) 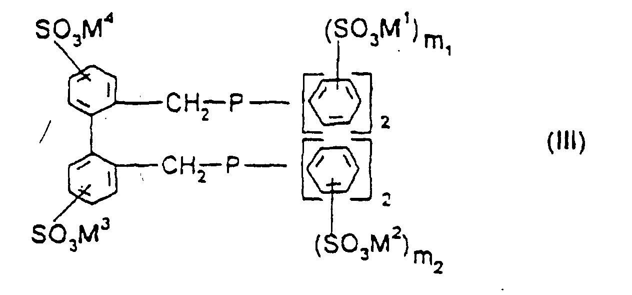 where
• m¹ and m² may be 0 or 1, and the sum of m¹ and m² is at least 1; and
• M¹, M², M³ and M⁴ are each, independently of one another, an alkaline metal ion or an ammonium ion.

9. A catalyst as claimed in claim 1, wherein the water-soluble sulfonated triarylphosphine is a compound of the formula (IV) 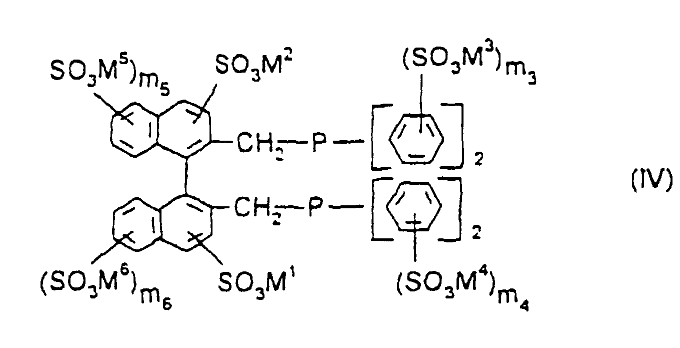 where
• m₃, m₄, m₅ and m₆ may be 0 or 1, and the sum of m₃, m₄, m₅ and m₆ is at least 2; and
• M¹, M², M³, M⁴, M⁵ and M⁶ are each, independently of one another, an alkaline metal ion or an ammonium ion.

10. A catalyst as claimed in claim 8 or 9 which contains from 20 to 500 ppm, preferably from 30 to 150 ppm, in particular from 40 to 100 ppm, of rhodium.

11. A catalyst as claimed in any one of claims 8 to 10, wherein the ratio of rhodium to the ligand is from 1 : 5 to 1 : 100, preferably from 1 : 5 to 1 : 50, in particular from 1 : 8 to 1 : 15.

12. A process for preparing aldehydes, which comprises reacting a C₉-C₁₈-olefin with hydrogen and carbon monoxide in the presence of a catalyst as claimed in any one of claims 1 to 11.

13. The process as claimed in claim 12, wherein the olefin is selected from among aliphatic, cycloaliphatic and araliphatic olefins, in particular from among aliphatic α-olefins and cycloaliphatic olefins.

14. The process as claimed in claim 12 or 13, wherein the olefin is selected from the group consisting of 1-nonene, 1-decene, 1-undecene, 1-dodecene, 1-tetradecene, 1-hexadecene and 1 -octadecene.

15. The process as claimed in any one of claims 12 to 14, wherein the olefin is present under the reaction conditions in a liquid phase which is immiscible with the catalyst.

16. The process as claimed in any one of claims 12 to 15, wherein the catalyst is used in an amount of from 2 × 10⁻⁶ to 5 × 10⁻² mol of rhodium per mol of olefinic compound.

17. The process as claimed in any one of claims 12 to 16, wherein the pressure during the reaction is from 20 to 150 bar, preferably from 30 to 80 bar.

18. The process as claimed in any one of claims 12 to 17, wherein the temperature during the reaction is from 50 to 150°C, preferably from 100 to 140°C.

## Revendications

1. Catalyseur contenant
(a) plus de 70% en poids d'un polyéthylèneglycol de formule (1)
H-(OCH₂CH₂)ₙ-OH (I)
dans laquelle
• n est un nombre entier entre 3 et 16 ; et
• la masse moléculaire moyenne en nombre est inférieure à 650 ;
(b) du rhodium sous forme élémentaire ou liée ;
(c) 2 à 25% en poids d'un ligand qui est une triarylphosphine sulfonée soluble dans l'eau ;
(d) au maximum 25% en poids d'eau.

2. Catalyseur selon la revendication 1, **caractérisé en ce que** le catalyseur contient au maximum 95% en poids du polyéthylèneglycol de formule (I).

3. Catalyseur selon la revendication 1 ou 2, **caractérisé en ce que** le catalyseur contient 80 à 85% en poids du polyéthylèneglycol de formule (I).

4. Catalyseur selon la revendication 1, **caractérisé en ce que** la triarylphosphine sulfonée soluble dans l'eau est une triarylphosphine sulfonée de formule (II) 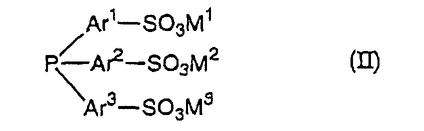 dans laquelle
• Ar¹, Ar² et Ar³ indépendamment l'un de l'autre représentent un groupe phényle, naphtyle, biphényle, phénylnaphtyle ou binaphtyle ;
• M¹, M² et M³ indépendamment l'un de l'autre représentent chaque lois un ion de métal alcalin ou un ion ammonium.

5. Catalyseur selon la revendication 1, **caractérisé en ce que** la triarylphosphine sulfonée est une triarylphosphine trisulfonée, en particulier la tri(m-sulfophényl)phosphine trisodique.

6. Catalyseur selon l'une des revendications précédentes, **caractérisé en ce que** le catalyseur contient 100 à 1000 ppm, de préférence 200 à 500 ppm, en particulier 300 à 400 ppm, de rhodium.

7. Catalyseur selon l'une des revendications précédentes, **caractérisé en ce que** le rapport du rhodium au ligand est entre 1 : 10 et 1 : 1000, de préférence entre 1 : 50 et 1 : 200.

8. Catalyseur selon la revendication, **caractérisé en ce que** la triarylphosphine sulfonée soluble dans l'eau est un composé de formule (III) 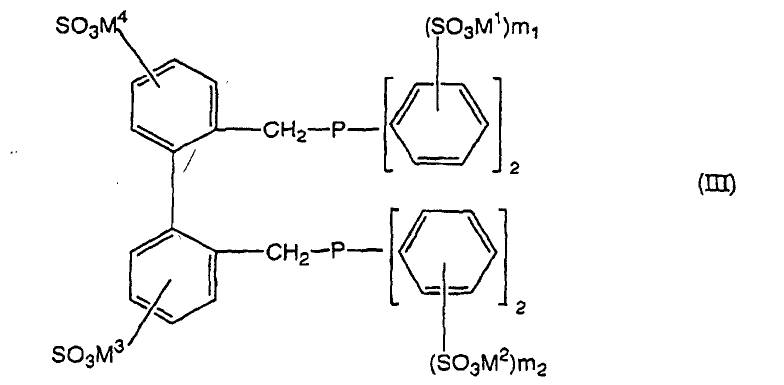 dans laquelle
• m¹ et m² peuvent avoir la valeur de 0 ou 1, avec la somme de m¹ et m² étant au moins 1, et
• M¹, M² M³ et M⁴ indépendamment l'un de l'autre représentent chacun un ion de métal alcalin ou un ion ammonium.

9. Catalyseur selon la revendication, **caractérisé en ce que** la triarylphosphine sulfonée soluble dans l'eau est un composé de formule (IV) 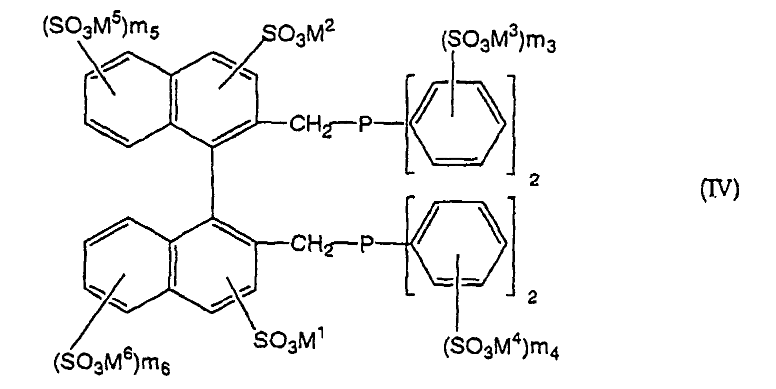 dans laquelle
• m₃, m₄, m₅ et m₆ peuvent avoir la valeur 0 ou 1, avec la somme de m₃, m₄, m₅ et m₆ étant au moins 2, et
• M¹, M² M³, M⁴, M⁵ et M⁶ indépendamment l'un de l'autre représentent chacun un ion de métal alcalin ou un ion ammønium.

10. Catalyseur selon la revendication 9, **caractérisé en ce qu'**il contient 20 à 500 ppm, de préférence 30 à 150 ppm, en particulier 40 à 100 ppm de rhodium.

11. Catalyseur selon l'une des revendications 8 à 10, **caractérisé en ce que** le rapport du rhodium au ligand est entre 1 : 5 et 1 : 100, de préférence entre 1 : 5 et 1 : 50, en particulier entre 1 : 8 et 1 : 15.

12. Procédé de préparation d'aldéhydes, **caractérisé en ce qu'**on fait réagir une oléfine en C₉-C₁₆ avec de l'hydrogène et du monoxyde de carbone en présence d'un catalyseur selon l'une des revendications 1 à 11.

13. Procédé selon la revendication 12, **caractérisé en ce que** l'oléfine est choisie parmi les oléfines aliphatiques, cycloaliphatiques et araliphatiques, en particulier des α-oléfines aliphatiques et des oléfines cycloaliphatiques.

14. Procédé selon la revendication 12 ou 13, **caractérisé en ce que** l'oléfine est choisie dans le groupe formé par le non-1-ène, le déc-1-ène, le undéc-1-ène, le dodéc-1-ène, le tétradéc-1-ène, le hexadéc-1-ène et le octadéc-1-ène.

15. Procédé selon l'une des revendications 12 à 14, **caractérisé en ce que** l'oléfine est présente dans des conditions réactionnelles dans une phase liquide, non miscible avec le catalyseur.

16. Procédé selon l'une des revendications 12 à 15, **caractérisé en ce que** le catalyseur est utilisé en une quantité correspondant à 2 x 10⁻⁶ à 5 x 10⁻² mole de rhodium par mole de composé oléfinique.

17. Procédé selon l'une des revendications 12 à 16, **caractérisé en ce que** la pression pendant la réaction est entre 20 et 150 bar, de préférence entre 30 et 80 bar.

18. Procédé selon l'une des revendications 12 à 17, **caractérisé en ce que** la température pendant la réaction est entre 50 et 150°C, de préférence entre 100 et 140°C.
